# EUROPEAN PATENT SPECIFICATION

(11) **EP 3 263 179 B1**
(45) Date of publication and mention of the grant of the patent: **19.02.2020**
(21) Application number: 16177330.4
(22) Date of filing: 30.06.2016
(51) Int. Cl.: A61Q 5/06, A61K 8/81, A61K 8/85

(54) **METHOD FOR STYLING HAIR FIBERS**
VERFAHREN ZUM STYLEN VON HAARFASERN
PROCÉDÉ PERMETTANT DE COIFFER DES FIBRES CAPILAIRES

(43) Date of publication of application: 03.01.2018
(73) Proprietor: Noxell Corporation, Hunt Valley, Maryland 21030-2098 (US)
(72) Inventor: KOCH, Malte, 65824 Schwalbach am Taunus (DE); FUCHS, Sybille, 65824 Schwalbach am Taunus (DE); BECKER, Martina, 65824 Schwalbach am Taunus (DE); FINDEIS, Bernd, 65824 Schwalbach am Taunus (DE); PRETZ, Kathrin, 65824 Schwalbach am Taunus (DE)
(74) Representative: Bandpay & Greuter

(56) References cited:
- WO-A2-2010/028142
- US-A1- 2002 155 962
- US-A1- 2016 175 238

## Description

### FIELD OF THE INVENTION

A method for styling hair fibers, preferably human hair fibers, comprises the following steps: providing a hair styling composition comprising, in a cosmetically acceptable carrier, a film-forming polymer; applying the hair styling composition to the hair fibers; heating the hair fibers comprising the hair styling composition at a temperature from 80°C to 230°C; setting the hair fibers in a hair style without subsequent rinsing; and cooling to room temperature.

Also, a kit for styling hair fibers, preferably human hair fibers, is provided and comprises an hair styling composition, preferably in a form of a hair styling gel or a non-aerosol hair spray; and an instructional material for preparation, for use, or both preparation and use, of the hair styling composition.

The hair styling composition can be used for immobilizing the hair temporarily and/or styling the hair in a thermoreversible manner.

### BACKGROUND OF THE INVENTION

The desire to have the hair retain a particular shape is widely used. The most common technology for accomplishing this is the application of a hair styling composition to dampened hair, after shampooing and/or conditioning, or to dry, styled hair. The hair styling compositions provide temporary setting benefits and they can be removed by water or by shampooing. The materials used in the hair styling compositions to provide the setting benefits have generally been resins and have been applied in the form of mousses, gels, lotions or sprays, see for instance International patent application WO 98/19653 A1.

Hair styling compositions comprise film-forming polymers used as fixatives in the form a lacquer or hair spray product for instance. See, for example, Ch. 30, Harry's Cosmeticology, 8th Ed., M. J. Rieger, Ph. D. (ed.), 666-667, Chemical Publishing Co., Inc., New York, NY (2000).

It is also known a method for styling hair, particularly comprising contacting the hair with a composition comprising a hair styling effective amount of a poly(vinylamine-vinylformamide). See WO 2010/028142 A2.

However, over the course of the day, the shape of the hair may rapidly disappear. Typically, the consumer needs to apply the hair styling composition again on his hair in order to recreate the initial shape.

Also, the hair styling composition typically uses fixative polymers which fix the hair in the final configuration when the fixative polymers are applied to the hair. However, often the fixative polymers do not display satisfying good adhesion to the hair fibers. Dusting or flaking may occur over extend periods of time or when the hair fibers are subjected to stresses such as brushing or combing.

Hence, there is a need to provide a method which can make it possible to easily shape the hair and to give it a desired relief with an improved shape-retention or at least a recoverable shape over time, while avoiding dusting or flaking off over time from the hair styling composition.

### SUMMARY OF THE INVENTION

A method for styling hair fibers, preferably human hair fibers, is provided and comprises the following steps:
(a) providing a hair styling composition comprising, in a cosmetically acceptable carrier, a film-forming polymer;
   wherein the hair styling composition comprises a total amount of film-forming polymer ranging from 2% to 60%, preferably from 5% to 50%, more preferably from 7% to 35%, even more preferably from 10% to 20% by total weight of the hair styling composition;
   wherein the film-forming polymer comprises one or more polymers as defined in the appended claims;
   wherein the film-forming polymer is water-soluble or water-dispersible at 25°C;
   wherein the film-forming polymer has a glass transition temperature from -20°C to 100°C; preferably from -20°C to 70°C, more preferably from 0°C to 65°C;
(b) applying the hair styling composition to the hair fibers;
(c) heating the hair fibers comprising the hair styling composition at a temperature from 80°C to 230°C, preferably from 100°C to 220°C, more preferably from 120°C to 180°C;
(d) setting the hair fibers in a hair style without subsequent rinsing;
   wherein the step (d) is performed subsequently after, simultaneously with or before the step (c); and
(e) cooling to room temperature.

Use of a hair styling composition for immobilizing the hair temporarily and/or styling the hair in a thermoreversible manner; wherein the hair styling composition comprises a total amount of film-forming polymer ranging from 2% to 60%, preferably from 5% to 50%, more preferably from 7% to 35%, even more preferably from 10% to 20% by total weight of the hair styling composition; wherein the film-forming polymer comprises one or more polymers as defined in the appended claims; and wherein the film-forming polymer has a glass transition temperature from - 20°C to 100°C; preferably from -20°C to 70°C, more preferably from 0°C to 65°C.

A kit for styling hair fibers, preferably human hair fibers, is provided and comprises an hair styling composition wherein the hair styling composition comprises a total amount of film-forming polymer ranging from 2% to 60%, preferably from 5% to 50%, more preferably from 7% to 35%, even more preferably from 10% to 20% by total weight of the hair styling composition; wherein the film-forming polymer comprises one or more polymers as defined in the appended claims; wherein the film-forming polymer is water-soluble or water-dispersible at 25°C; and wherein the film-forming polymer has a glass transition temperature from -20°C to 100°C; preferably from - 20°C to 70°C, more preferably from 0°C to 65°C, preferably in a form of a hair styling gel or a non-aerosol hair spray; and an instructional material for preparation, for use, or both preparation and use, of the hair styling composition; and optionally an appliance for heating and setting the hair fibers in hair style, wherein the appliance is selected from the group consisting of a flat iron, a round iron, a curling iron, a straightening iron and a waving iron.

### DETAILED DESCRIPTION OF THE INVENTION

### Definitions of terms

In this document, including in all embodiments of all aspects of the present invention, the following definitions apply unless specifically stated otherwise.

All percentages are by weight (w/w) of the respective composition, unless otherwise specified. All ratios or percentages are weight ratios or weight percentages unless specifically stated otherwise. "% wt." means percentage by weight. References to "parts" e.g. a mixture of 1 part X and 3 parts Y, is a ratio by weight. When more than one composition are used during a treatment, the total weight to be considered is the total weight of all the compositions applied on the hair simultaneously (i.e. the weight found "on head"), unless otherwise specified.

"QSP" means sufficient quantity for 100% or for 100g. "+/-" indicates the standard deviation. All ranges are inclusive and combinable. The number of significant digits conveys neither a limitation on the indicated amount nor on the accuracy of the measurement. All numerical amounts are understood to be modified by the word "about".

All measurements are understood to be made at 25°C and at ambient conditions, where "ambient conditions" means at 1 atmosphere (atm) of pressure and at 65% relative humidity, unless otherwise stated. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. "Room temperature" means 25°C.

Herein "min" means "minute" or "minutes". Herein "mol" means mole. Herein "g" following a number means "gram" or "grams". "Ex." means "example". All amounts as they pertain to listed ingredients are based on the active level ("solids") and do not include carriers or by-products that may be included in commercially available materials.

Herein, "comprising" means that other steps and other ingredients can be in addition. "Comprising" encompasses the terms "consisting of' and "consisting essentially of'. The compositions, methods, uses, and kits of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the composition.

The term "substantially free of" as used herein means less than 1%, less than 0.8%, less than 0.5%, less than 0.3%, or less than an immaterial amount of by total weight of the composition.

The term "hair" as used herein means mammalian hair including scalp hair, facial hair and body hair, more preferably hair on the human head and scalp. Hair comprises hair fibers. "Hair shaft" means an individual hair strand and may be used interchangeably with the term "hair." As used herein the term "hair" to be treated may be "living" i.e. on a living body or may be "non-living" i.e. in a wig, hairpiece or other aggregation of non-living keratinous fibers. Mammalian, preferably human hair is preferred.

The term "film-forming polymer" as used herein means a polymer which is in a position to deposit a polymer film on the hair.

The term "copolymer" as used herein refers to a polymer derived from two or more polymerizable monomers. When used in generic terms, the term "copolymer" is also inclusive of more than two distinct monomers, for example, ter-polymers.

The term "temporary hair styling" means a hair style of human hair which lasts until the next hair washing.

The term "cosmetically acceptable" as used herein means that the compositions, or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

The term "kit" as used herein means a packaging unit comprising a plurality of components i.e. a kit of parts. An example of a kit is, for example, a first composition, a separately packaged second composition and a separately packaged third composition. Another kit may comprise application instructions comprising a method and a first, second and third compositions.

### Method for styling hair fibers

A method for styling hair fibers, preferably human hair fibers, comprises the following steps: providing a hair styling composition comprising, in a cosmetically acceptable carrier, a film-forming polymer; applying the hair styling composition to the hair fibers; heating the hair fibers comprising the hair styling composition at a temperature from 80°C to 230°C; setting the hair fibers in a hair style without subsequent rinsing; and cooling to room temperature.

The application of the hair styling composition to the hair fibers is relatively easy as detailed more below. The film-forming polymer can deposit on the hair fibers with an excellent adhesion. The cosmetic feel of the hair fibers treated according to the method of the present invention can be very good.

It has been found that when bending the hair fibers treated according to the method of the present invention to a certain extent, the group of hair fibers united by the film-forming polymer tends to come apart from each other, however without any dusting or flaking off from the hair styling composition.

Without wishing to be bound by theory, it is believed that the cohesion of the hair fibers welded together by the film-forming polymer is weaker than the adhesion of the film-forming polymer to the hair fibers. In other words, when bending the hair fibers, the bonds between the film-forming polymer and the hair fibers welded together by the film-forming polymer break before the bonds between each single hair fiber and the film-forming polymer. In that case, the film-forming polymer remains on each hair fiber as a relatively thin film deposit. Hence, when the hair style has disappeared or has moved, it is possible to reshape the hair fibers together to the desired relief by heating the hair fibers above the glass transition temperature of the film-forming polymer without adding any additional amount of the film-forming polymer. The method allows providing a thermoreversible shaping of the hair fibers coated with the film-forming polymer of the hair styling composition.

### Hair styling composition

The hair styling composition comprises, in a cosmetically acceptable carrier, a film-forming polymer. The hair styling composition comprises a total amount of film-forming polymer ranging from 2% to 60%, preferably from 5% to 50%, more preferably from 7% to 35%, even more preferably from 10% to 20% by total weight of the hair styling composition.

The film-forming polymer is water-soluble or water-dispersible at 25°C. The term "water-soluble or water-dispersible" means that the film-forming polymer which, when introduced into an aqueous phase at 25°C, to a mass concentration equal to 1%, make it possible to obtain a macroscopically homogeneous and transparent solution, i.e. a solution that has a maximum light transmittance value, at a wavelength equal to 500 nm, through a sample 1 cm thick, of at least 60% and preferably of at least 70%.

The film-forming polymer has a glass transition temperature (Tg) from -20°C to 100°C; preferably from -20°C to 70°C, more preferably from 0°C to 65°C, even more preferably from 0°C to 35°C. The glass transition temperature can be measured by differential scanning calorimetry (DSC) according to ASTM D3418-97.

Film-forming polymers may comprise one or more polymers, such as polyesters or acrylic copolymers, selected from block, graft, branched and cross-linked copolymers derived from two or more monomers selected from the group consisting of (meth)acrylic acid, crotonic acid, itaconic acid, citraconic acid, aconitic acid, (C₁-C₄-alkyl)-ester of (meth)acrylic acid, (C₁-C₄-hydroxyalkyl)-ester of (meth)acrylic acid, (meth)acrylamide, N-(C₁-C₄-alkyl)(meth)acrylamide, maleic acid, fumaric acid, aryl-dicarboxylic acid, aryl-acid anhydride, (C₁-C₄-alkyl)-ester of aryl-dicarboxylic acid, aryl-sulfonated dicarboxylic acid, aryl-sulfonated acid anhydride, (C₁-C₄-alkyl)-ester of aryl-sulfonated dicarboxylic acid, and mixtures thereof. (C₁-C₄-alkyl) may be either methyl, or ethyl, or propyl, or isopropyl, or n-butyl, or sec-butyl, or tertio-butyl. (C₁-C₄-hydroxyalkyl) may be hydroxymethyl, or hydroxyethyl, or hydroxypropyl, or hydroxybutyl.

Film-forming polymers may comprise one or more polyesters selected from copolymers derived from two or more monomers selected from the group consisting of isophthalic acid, terephthalic acid, sulfonated-isophthalic acid, sulfonated-terephthalic acid, (C₁-C₄-alkyl)-ester of sulfonated-isophthalic acid, (C₁-C₄-alkyl)-ester of sulfonated-terephthalic acid, and mixtures thereof; or one or more acrylic copolymers derived from two or more monomers selected from the group consisting of (meth)acrylic acid, (C₁-C₄-alkyl)-ester of (meth)acrylic acid, (C₁-C₄-hydroxyalkyl)-ester of (meth)acrylic acid, and mixtures thereof.

Film-forming polymers may comprise one or more polyesters which are aryl-sulfonated polyesters selected from copolymers derived from two or more monomers selected from the group consisting of isophthalic acid, terephthalic acid, 5-sulfonated-isophthalic acid, 5-sulfonated-terephthalic acid, (C₁-C₄-alkyl)-ester of 5-sulfonated-isophthalic acid, (C₁-C₄-alkyl)-ester of 5-sulfonated-terephthalic acid, and mixtures thereof; or one or more acrylic copolymers derived from two or more monomers selected from the group consisting of (meth)acrylic acid, (C₁-C₄-alkyl)-ester of (meth)acrylic acid, (C₁-C₄-hydroxyalkyl)-ester of (meth)acrylic acid, and mixtures thereof.

Film-forming polymers may comprise one or more acrylic copolymers derived from two or more monomers selected from the group consisting of (meth)acrylic acid, (C₁-C₄-alkyl)-ester of (meth)acrylic acid, (C₁-C₄-hydroxyalkyl)-ester of (meth)acrylic acid, and mixtures thereof.

Polyesters, preferably the aryl-sulfonated polyesters and the acrylic copolymers have both the common property to form a relatively thin film deposit on the hair fibers. Both polyesters, preferably the aryl-sulfonated polyesters and the acrylic copolymers exhibit thermoreversible properties. Hence, when the hair fibers coated by one of these film-forming polymers are heated at a temperature above the glass transition of the respective film-forming polymer, the hair fibers can be readily shaped in a thermoreversible manner.

The aryl-sulfonated polyesters and the acrylic copolymers, according to the present invention and as defined in the appended claims, are defined below.

### Aryl-sulfonated polyesters

Film-forming polymers may be aryl-sulfonated polyesters derived from two or more monomers selected from the group consisting of aryl-dicarboxylic acid, aryl-acid anhydride, (C₁-C₄-alkyl)-ester of aryl-dicarboxylic acid, aryl-sulfonated dicarboxylic acid, aryl-sulfonated acid anhydride, (C₁-C₄-alkyl)-ester of aryl-sulfonated dicarboxylic acid, and mixtures thereof. (C₁-C₄-alkyl) may be either methyl, or ethyl, or propyl, or isopropyl, or n-butyl, or sec-butyl, or tertio-butyl.

Film-forming polymers may comprise one or more polyesters selected from copolymers derived from two or more monomers selected from the group consisting of isophthalic acid, terephthalic acid, sulfonated-isophthalic acid, sulfonated-terephthalic acid, (C₁-C₄-alkyl)-ester of sulfonated-isophthalic acid, (C₁-C₄-alkyl)-ester of sulfonated-terephthalic acid, and mixtures thereof.

Film-forming polymers may comprise one or more polyesters selected from copolymers derived from two or more monomers selected from the group consisting of isophthalic acid, terephthalic acid, 5-sulfonated-isophthalic acid, 5-sulfonated-terephthalic acid, (C₁-C₄-alkyl)-ester of 5-sulfonated-isophthalic acid, (C₁-C₄-alkyl)-ester of 5-sulfonated-terephthalic acid, and mixtures thereof.

The term "aryl-sulfonated polyester" means copolyesters obtained by polycondensation of at least one dicarboxylic acid or of an ester thereof, of at least one diol and of at least one sulfoaryldicarboxylic difunctional compound substituted on the aromatic nucleus with a sulfonated group -SO3M in which M represents a hydrogen atom, an ammonium ion, substituted ammonium or a metal ion such as Na⁺, Li⁺ or K⁺.

The aryl-sulfonated polyesters may generally have a weight-average molecular mass of between 1 000 Da and 60 000 Da, preferably from 5 000 Da to 40 000 Da, more preferably from 20 000 Da to 30 000 Da as determined by gel permeation chromatography (or GPC).

The glass transition temperature (Tg) of these aryl-sulfonated polyesters is within the range from -20°C to 100°C, preferably within the range from 50°C to 100°C and preferably from 50°C to 70°C.

The aryl-sulfonated polyesters are described in greater detail in the following patent applications U.S. Pat. No. 3,734,874; U.S. Pat. No. 3,779,993; U.S. Pat. No. 4,119,680; U.S. Pat. No. 4,300,580; U.S. Pat. No. 4,973,656; U.S. Pat. No. 5,660,816; U.S. Pat. No. 5,662,893; and U.S. Pat. No. 5,674,479.

According to the present invention, the aryl-sulfonated polyester is a polycondensate of at least one dicarboxylic acid or of an ester thereof, of at least one diol and of at least one 5-sulfoaryldicarboxylic difunctional compound substituted on the aromatic nucleus with a sulfonated group - SO₃M in which M represents a hydrogen atom, an ammonium ion, substituted ammonium or a metal ion such as Na⁺, Li⁺ or K⁺. This aryl-sulfonated polyester is obtained from isophthalic acid, from the sodium salt of sulfoisophthalic acid, from diethylene glycol and from 1,4-cyclohexane-methanol. The aryl-sulfonated polyester preferably used in the invention may comprise at least units derived from isophthalic acid, from a salt of 5-sulfoaryldicarboxylic acid and from diethylene glycol, and more particularly, the aryl-sulfonated polyester used in the invention are obtained from isophthalic acid, from the sodium salt of 5-sulfoisophthalic acid, from diethylene glycol and from 1,4-cyclohexanemethanol.

The aryl-sulfonated polyesters may be prepared by melt-phase polymerization of glycols and aromatic diacids. Typical monomers used to produce the aryl-sulfonated polyesters are isophthalic acid or terephthalic acid, 5-sodium (or 5-potassium, or 5-lithium)-sulfoisophthalic acid, 5-sodium (or 5-potassium, or 5-lithium)-sulfoterephthalic acid, 1,4-cyclohexanedimethanol, and diethylene glycol.

Examples of aryl-sulfonated polyester that may especially be mentioned include those known under the INCI name Polyester-5 and sold under the trade name "Eastman AQ™ polymer" (by the company Eastman Chemical). Eastman AQ™ 38S, 48 Ultra, or 55S are non-limited examples of aryl-sulfonated polyesters that can be used in the hair styling composition of the present invention. They contain the four following monomers isophthalic acid, 5-sodium-sulfoisophthalic acid, 1,4-cyclohexanedimethanol, and diethylene glycol, but in different ratios.

As described hereinafter, an Example C1 of the hair styling composition comprising Eastman AQ™ 38S polymer as the film-forming polymer has been prepared and used according to the method of the present invention. Eastman AQ™ 38S polymer has a glass transition temperature of 38°C.

Also, an Example C2 of the hair styling composition comprising Eastman AQ™ 2350 polymer as the film-forming polymer has been prepared and used according to the method of the present invention. Eastman AQ™ 2350 polymer has a glass transition temperature of 10°C

Both Eastman AQ™ 38S polymer and Eastman AQ™ 2350 polymer contain isophthalic acid, 5-sodiosulfoisophthalic acid, 1,4-cyclohexanedimethanol, and diethylene glycol, but in different ratios.

The hair styling composition may comprise a total amount of aryl-sulfonated polyester ranging from 2% to 60%, preferably from 5% to 50%, more preferably from 7% to 35%, even more preferably from 10% to 20% by total weight of the hair styling composition.

Unlike solution polymers whose chains entangle at low concentration, aryl-sulfonated polyester dispersions have a relatively low viscosity. The viscosity can rise when increasing concentration of the aryl-sulfonated polyester in the hair styling composition.

Hence, the hair styling composition may comprise a total amount of aryl-sulfonated polyester ranging from 2% to 60%, by total weight of the hair styling composition and the hair styling composition may have a viscosity of from 25 centipoises to 30 000 centipoises according to the Viscosity Test Method.

The hair styling composition may preferably comprise a total amount of aryl-sulfonated polyester ranging from 10% to 20%, by total weight of the hair styling composition and the hair styling composition may have a viscosity of from 50 centipoises to 3 000 centipoises according to the Viscosity Test Method.

Aryl-sulfonated polyesters in aqueous dispersions exhibit good chemical stability at a pH ranging from 5 to 8. Hence, the hair styling composition may have a pH from 5 to 8, preferably from 6 to 7.5.

### Acrylic copolymers

Film-forming polymers may be acrylic copolymers. The film-forming polymer of the hair styling composition is a crosslinked acrylic copolymer which is obtained by polymerization from a monomer composition comprising three different monomer components, a1), b1), c1), and an optional fourth monomer component d).

The first monomer component a1) may be selected from one or more monoethylenically unsaturated monomers containing at least one carboxylic acid group. The first monomer component a1) may be selected from the group consisting of (meth)acrylic acid, itaconic acid, citraconic acid, maleic acid, fumaric acid, crotonic acid, aconitic acid, and mixtures thereof. According to the present invention, the first monomer component a1) is (meth)acrylic acid.

The amount of the at least one carboxylic acid group containing monomer set forth under the first monomer component a1) may range from 10% to 80% by weight, preferably from 20% to 70% by weight, more preferably from 35% to 65% by weight, based upon the total weight of the monomers in the polymerizable monomer composition.

The second monomer component b1) may be selected from at least one monoethylenically unsaturated monomer containing at least one linear or branched C₁ to C₄ alkyl ester of (meth)acrylic acid, at least one C₁ to C₄ hydroxyalkyl ester of (meth)acrylic acid, and mixtures thereof. According to the present invention, the second monomer component b1) is selected from at least one monoethylenically unsaturated monomer containing at least one linear or branched C₁ to C₄ alkyl ester of (meth)acrylic acid, and mixtures thereof.

Suitable exemplary linear or branched C₁ to C₄ alkyl (meth)acrylate and hydroxyalkyl (meth)acrylate monomers set forth under the second monomer component b1) include methyl (meth)acrylate, ethyl (meth)acrylate, n-propyl (meth)acrylate, iso-propyl (meth)acrylate, n-butyl (meth)acrylate, isobutyl (meth)acrylate, t-butyl (meth)acrylate, hydroxyethyl (meth)acrylate, hydroxypropyl (meth)acrylate, hydroxybutyl (meth)acrylate (butane diol mono(meth)acrylate), and mixtures thereof.

The monomers set forth under the second monomer component b1) may be utilized in an amount ranging from 90% to 15% by weight, preferably from 80% to 25% by weight, more preferably from 65% to 35% by weight, based upon the total weight of the monomers in the polymerizable monomer composition.

The third monomer component c1) comprises a mixture of crosslinking monomers selected from at least two different polyunsaturated monomer types or classes. The first crosslinking monomer is selected from at least one polyfunctional acrylate having at least two polymerizable ethylenically unsaturated double bonds. The term "polyfunctional acrylate" refers to acrylate esters of organic polyols wherein the organic polyol is esterified by reacting it with (meth)acrylic acid. The polyfunctional acrylate can contain from 2 to 6 polymerizable ethylenically unsaturated double bonds. Suitable polyols for the esterification reaction can contain from 2 to 12 carbon atoms and have at least two hydroxyl groups. The polyol can be linear and branched or cyclic.

Exemplary polyols suitable for esterification may include, but are not limited to, alkylene glycols containing from 2 to 5 carbon atoms, polyalkylene glycol dimers and trimers, trimethylolethane and dimers thereof, trimethylolpropane and dimers thereof, triethylolpropane and dimers thereof, tetramethylolmethane (pentaerythritol), dipentaerythritol, and 1,4-cyclohexanediol.

Exemplary polyfunctional acrylates include, but are not limited to, ethylene glycol di(meth)acrylate, triethylene glycol di(meth)acrylate, 1,3-butylene glycol di(meth)acrylate, 1,4-butylene glycol di(meth)acrylate, 1,6-hexanediol di(meth)acrylate, neopentyl glycol di(meth)acrylate, 1,9-nonanediol di(meth)acrylate, trimethylolpropane tri(meth)acrylate, trimethylolethane tri(meth)acrylate, tetramethylolmethane tri(meth)acrylate, ditrimethylolpropane tetra(meth)acrylate, tetramethylolmethane tetra(meth)acrylate, pentaerythritol tetra(meth)acrylate; dipentaerythritol hexa(meth)acrylate, 1,4-cyclohexanediol dimethyacrylate, and mixtures thereof.

The second crosslinking monomer may be selected from at least one polyalkenyl polyether having at least two polymerizable ethylenically unsaturated double bonds. The term "polyalkenyl polyether" refers to alkenyl ethers of organic polyols wherein the organic polyol is etherified by reacting it with alkenyl halide, such as allyl chloride or allyl bromide. The polyalkenyl polyether (e.g., polyallyl polyether) may contain from 2 to 8 polymerizable ethylenically unsaturated double bonds. Suitable polyols for the etherification reaction may contain from 2 to 12 carbon atoms and have at least two hydroxyl groups. The polyol may be linear and branched or cyclic (e.g., monosaccharides and polysaccharides containing 1 to 4 saccharide units).

Exemplary polyols suitable for etherification may include, but are not limited to, glucose, galactose, fructose, sorbose, rhamnose, sucrose, arabinose, maltose, lactose, raffinose, pentaerythritol, dipentaerythritol, trimethylolethane and dimers thereof, trimethylolpropane and dimers thereof, and triethylolpropane and dimers thereof. Additional polyols suitable for the etherification are disclosed in U. S. Patent No. 2,798,053.

Exemplary polyalkenyl polyethers may include, but are not limited to, polyallyl ethers of sucrose having from 2 to 8 allyl groups per molecule, pentaerythritol diallyl ether, pentaerythritol triallyl ether, and pentaerythritol tetraallyl ether; trimethylolpropane diallyl ether, trimethylolpropane triallyl ether, and mixtures thereof.

The mixture of crosslinking monomers (first and second crosslinking monomer components) set forth under the third monomer component c1) may be present in an amount ranging from 0.01% to 5% by weight, preferably from 0.03% to 3% by weight, more preferably from 0.05% to 1 % by weight, based upon the total weight of the monomers in the polymerizable monomer composition.

The weight ratio of the first crosslinking monomer to the second crosslinking monomer in the polymerizable monomer mixture may range from 1:99 to 99:1, preferably from 1:9 to 9:1, more preferably from 2:8 to 8:2, more preferably from about 4:6 to about 6:4.

The optional fourth monomer component d1) may be a monoethylenically unsaturated monomer different from monomer components a1), b1), and c1).

Exemplary ethylenically unsaturated monomers of the optional fourth monomeric component d1) may include ethyl diglycol (meth)acrylate, 2-carboxyethyl (meth)acrylate, n-hexyl (meth)acrylate, 2-ethylhexyl (meth)acrylate, octyl (meth)acrylate, decyl (meth)acrylate, 6-hydroxyhexyl (meth)acrylate, 10-hydroxydecyl (meth)acrylate, styrene, α-methyl styrene, acrylonitrile, methacrylonitrile, acrylamide, methacrylamide, N,N'-dimethylaminoacrylamide, t-butylacrylamide, t-octylacrylamide, N-vinyl pyrrolidone, 2-acrylamido-2-methylpropane sulfonic acid, vinyl acetate, vinyl propionate, vinyl butanoate, vinyl valerate, vinyl hexanoate, vinyl octanoate, vinyl nonanoate, vinyl decanoate, vinyl neodecanoate, vinyl undecanoate, vinyl laurate, ACE(TM) and (M)ACE(TM) monomer available from Hexion Specialty Chemicals, Inc., Columbus, OH; and mixtures thereof. The foregoing monomers are commercially available and/or can be synthesized by procedures well known in the art, or as described herein.

As one of ordinary skill in the art will readily recognize the amounts of each of the first, second, third, and optional fourth monomer components (a1-d1) set forth herein will be selected from the disclosed ranges such that the sum of each of the monomer components in the polymerizable monomer compositions is equal to 100 wt.%.

Hence, the film-forming polymer of the hair styling composition may be a crosslinked acrylic copolymer which is obtained by polymerization from a monomer composition comprising
- a first monomer component a1) being (meth)acrylic acid;
- a second monomer component b1) selected from at least one monoethylenically unsaturated monomer containing at least one linear or branched C₁ to C₄ alkyl ester of (meth)acrylic acid, and mixtures thereof;
- a third monomer component c1) comprising a mixture of crosslinking monomers selected from a first and second crosslinking monomer, wherein the first crosslinking monomer is selected from at least one polyfunctional acrylate having at least two polymerizable ethylenically unsaturated double bonds, and the second crosslinking monomer is selected from at least one polyalkenyl polyether having at least two polymerizable ethylenically unsaturated double bonds; and
- an optional fourth monomer component d1), which is a monoethylenically unsaturated monomer different from the first, second and third monomer components a1), b1), and c1).

An example of an acrylic copolymer for the hair styling composition may especially be the acrylic polymer known under the INCI name Polyacrylate-14 and sold under the trade name Fixate™ PLUS polymer, which is a slightly crosslinked acrylic copolymer comprising at least (meth)acrylic acid and C₁₋₄ alkyl(meth)acrylate, as the respective first and second monomer components a1) and b1).

The hair styling composition may comprise a total amount of acrylic copolymer ranging from 2% to 60%, preferably from 5% to 50%, more preferably from 7% to 35%, even more preferably from 10% to 20% by total weight of the hair styling composition.

The hair styling composition may comprise a total amount of acrylic copolymer ranging from 2% to 60%, by total weight of the hair styling composition and the hair styling composition may have a viscosity of from 0,025 Pa.s to 30 Pa.s according to the Viscosity Test Method.

The hair styling composition may preferably comprise a total amount of acrylic copolymer ranging from 10% to 20%, by total weight of the hair styling composition and the hair styling composition may have a viscosity of from 0,05 Pa.s to 3 Pa.s according to the Viscosity Test Method.

As described hereinafter, an Example C3 of the hair styling composition comprising Fixate™ PLUS polymer as the film-forming polymer has been prepared and used according to the method of the present invention. Fixate™ PLUS polymer has a glass transition temperature of 63 °C

### Optional features for the hair styling composition

The hair styling composition may have a viscosity of from 25 centipoises to 30 000 centipoises, preferably from 30 centipoises to 10 000 centipoises, more preferably from 40 centipoises to 5 000 centipoises, even more preferably from 50 centipoises to 3 000 centipoises according to the Viscosity Test Method. Indeed, the hair styling composition having the viscosity as stated herein before can be non-runny in order to avoid dropping from opened and inverted products containers and from the fingers or any application forms used to apply the hair styling composition to the hair fibers. The skilled person will know how to choose the sufficient viscosity by increasing the amount of the film-forming polymer in the hair styling composition, for instance. The method of the present invention may be carried out with a liquid hair styling composition.

The hair styling composition may have a pH from 5 to 8, preferably from 6 to 7.5. The hair styling composition may be provided in a cosmetically acceptable carrier such as water, or an alcohol such as ethanol or a mixture of ethanol and water. It is intended that the hair styling composition may preferably have a neutral pH to avoid any hydrolysis of the film-forming polymer, or any precipitation.

The hair styling composition may be substantially free of surfactants, or may comprise a total amount of surfactants ranging from 1 % to 10%, preferably from 1.5% to 5% by total weight of the hair styling composition. Having the hair styling composition free of surfactants can help to prevent the film-forming polymer to swell.

### Optional ingredients for the hair styling composition

The hair styling composition may also contain one or more additives chosen from nonionic, anionic, cationic and amphoteric polymers, ceramides and pseudo ceramides, vitamins and provitamins including panthenol, water-soluble and liposoluble, silicone or non-silicone sunscreens, pigments, nacreous agents and opacifiers, active particles, dyes or coloured materials, sequestering agents, plasticizers, solubilizers, acidifying agents, basifying agents, neutralizers, thickeners, antioxidants, hydroxy acids, penetrating agents, perfume and preservatives.

A person skilled in the art will take care to select the optional additives and the amount thereof such that they do not harm the properties of the hair styling compositions according to the present invention.

These additives may be present in the hair styling composition according to the invention in an amount ranging from 0% to 20% by weight relative to the total weight of the composition.

The hair styling composition may further comprise one or more pigments or one or more colored materials. Pigments or coloured materials can help to provide a nice glow effect for the hair fibers setting by the application of the hair styling composition and the heating step.

### Pigment

The hair styling composition may comprise one or more pigments. The one or more pigments of the hair styling composition may be a colored pigment which imparts color effects to the composition or to the hair.

Alternatively, the one or more pigments of the hair styling composition may be a luster effect pigment which imparts desirable and aesthetically pleasing luster effects to the composition or to the keratin fibers of the hair. The color or luster effects on the keratin fibers of the hair may be preferably temporary. Indeed, the color or luster effects on the keratin fibers of the hair can last until the next hair wash and can be removed again by washing the hair with customary shampoos.

The hair styling composition may be substantially free of pigment. Indeed, having a hair styling composition substantially free of pigment can help to prevent the formation of residues, precipitation and/or rough hair feel.

The hair styling composition may comprise from 0.01% to 25%, or from 0.1% to 20%, or from 1% to 15%, or from 4% to 10% of the one or more pigments by total weight of the hair styling composition.

The one or more pigments of the hair styling composition may be a colorant which is virtually insoluble in the composition, and may be inorganic or organic. Inorganic-organic mixed pigments may be also possible. The hair styling composition may comprise an inorganic pigment. The advantage of an inorganic pigment is its excellent resistance to light, weather and temperature. The inorganic pigment of the hair styling composition may be of natural origin, and may be, for example, derived from a material selected from the group consisting of chalk, ochre, umber, green earth, burnt sienna, and graphite.

The one or more pigments of the hair styling composition may be a white pigment, such as, for example, titanium dioxide or zinc oxide. Alternatively, the one or more pigments of the hair styling composition may be a black pigment, such as, for example, iron oxide black. Alternatively, the one or more pigments of the hair styling composition may be a colored pigment, such as, for example, ultra-marine or iron oxide red, or a luster pigment, or a metal effect pigment, or a pearlescent pigment, and/or a fluorescent or phosphorescent pigment.

The one or more pigments of the hair styling composition may be selected from the group consisting of metal oxides, hydroxides and oxide hydrates, mixed phase pigments, sulfur-containing silicates, metal sulfides, complex metal cyanides, metal sulfates, chromates and molybdates, the metals themselves (bronze pigments), and combinations thereof. The one or more pigments of the hair styling composition may be selected from the group consisting of titanium dioxide (CI 77891), black iron oxide (CI 77499), yellow iron oxide (CI 77492), red and brown iron oxide (CI 77491), manganese violet (CI 77742), ultramarine (sodium aluminium sulfosilicates, CI 77007, Pigment Blue 29), chromium oxide hydrate (CI 77289), Prussian blue (ferric ferrocyanide, CI 77510), carmine (cochineal), and combinations thereof.

The one or more pigments of the hair styling composition may be a synthetic organic pigment. The synthetic organic pigment of the hair styling composition may be selected from the group consisting of azo pigments, anthraquinoids, indigoids, dioxazine, quinacridone, phthalocyanine, isoindolinone, perylene and perinone, metal complex, alkali blue, diketopyrrolopyrrole pigments, and combinations thereof.

The one or more pigments of the hair styling composition may be selected from the group consisting of iron oxide, titanium dioxide, mica, borosilicate, and combinations thereof. The pigment of the hair styling composition may comprise an iron oxide (Fe₂O₃) pigment. The one or more pigments of the hair styling composition may comprise a combination of mica and titanium dioxide.

### Colored material

The hair styling composition may comprise one or more colored materials. The one or more colored materials of the hair styling composition may be particulate in form. The one or more colored materials of the hair styling composition may be selected from the group consisting of colored fibers, colored beads, colored particles such as nano-particles, colored polymers comprising covalently attached dyes, liquid crystals, particles having diffraction properties, UV absorber and photoprotective substances, pressure- or light-sensitive pigments, and combinations thereof.

The hair styling composition may be substantially free of colored material. Indeed, having a hair styling composition substantially free of colored material can help to prevent the formation residues and precipitation.

The one or more colored materials of the hair styling composition may be capable of changing color via a mechanism selected from the group consisting of thermochromism, photochromism, hydrochromism, magnetochromism, electrochromism, piezochromism, chemichromism, mechano-optics. Suitable colored materials of the hair styling composition may include 3D Magnetic Pigments, Glow Dust, Fluorescent Pigments, Thermo Dust, Chameleon Pigments and other color changing materials from Solar Color Dust (http://solarcolordust.com/).

The hair styling composition may comprise from 0.01% to 10%, or from 0.05% to 5% of one or more particulate substances by total weight of the hair styling composition. The one or more particulate substances of the hair styling composition may be selected from the group consisting of silica, silicates, aluminates, clay earths, mica, and insoluble salts. The one or more particulate substances of the hair styling composition may be selected from the group consisting of insoluble inorganic metal salts, metal oxides, minerals and insoluble polymer particles. The one or more particulate substances of the hair styling composition may be titanium dioxide.

Alternatively, the one or more particulate substances of the hair styling composition may be selected from the group consisting of metal salts such as alkali metal or alkaline earth metal halides, e.g. sodium chloride or potassium chloride; alkali metal or alkaline earth metal sulfates, such as sodium sulfate or magnesium sulfate.

### Thickeners

The hair styling composition according to the invention may further comprise one or more thickeners in an amount sufficient to provide the hair styling composition with a viscosity so that it can be readily applied to the hair without unduly dripping off the hair and causing mess.

Suitable thickeners include, but are not limited to: associative polymers, polysaccharides, non-associative polycarboxylic polymers, and mixtures thereof.

Typically, the hair styling composition may comprise a total amount of thickeners ranging from at least 0.1%, preferably at least 0.5%, more preferably at least 1%, by total weight of the hair styling composition.

### Cosmetically acceptable carrier

The hair styling composition comprises a cosmetically acceptable carrier. The cosmetically acceptable carrier of the hair styling composition may be an aqueous carrier. The composition may comprise water. The hair styling composition may comprise from 50% to 85% water, or from 65% to 75% of water by total weight of the composition.

The cosmetically acceptable carrier is any carrier suitable for formulating the film-forming polymer into a hair styling composition being suitable for application onto hair. The cosmetically acceptable carrier may be selected from either an aqueous medium or an aqueous-alcoholic medium. When the cosmetically acceptable carrier is an aqueous-alcoholic carrier, the cosmetically acceptable carrier may comprise water and an alcohol. An alcohol can advantageously influence the viscosity of a relatively wide spectrum of ingredients of the composition. The alcohol of the composition may be selected from the group consisting of ethanol, isopropanol, propanol, and mixtures thereof.

When the cosmetically acceptable carrier is an aqueous carrier, the aqueous carrier may consist essentially of water and may be substantially free of alcohol. The composition may comprise a safe and effective amount of a cosmetically acceptable carrier which is water.

When used daily, organic solvents in hair styling compositions may have a negative effect on humans and on the environment. They also have an unpleasant odor. Hence, the hair styling composition may be substantially free of organic solvents. Organic solvents may be any volatile alcohols, e.g. ethanol, isopropanol, propanol.

### Preservative

The hair styling composition may comprise at least one preservative and/or a mixture of preservatives. The hair styling composition may comprise from 0.01% to 1% preservative, or from 0.1% to 0.5% preservative by total weight of the hair styling composition. The preservative of the hair styling composition may be selected from the group consisting of benzyl alcohol, phenoxyethanol, 1,3-bis(hydroxymethyl)-5,5-dimethylimidazolidine-2,4-dione, and mixtures thereof. The hair styling composition may be substantially free of benzoate compounds. Indeed, having benzoate compounds can help to prevent instability and/or precipitation of the hair styling composition. The hair styling composition may be substantially free of parabens.

### Perfume

The hair styling composition may comprise a perfume. The composition may comprise from 0.001% to 2% of a perfume by total weight of the hair styling composition. Perfume can provide an enhanced user experience by making the composition smell pleasant and/or invoke emotions tailored to the visual effects on the hair fibers, such as relaxing or exciting smells.

Alternatively, the hair styling composition may be substantially free of perfume and/or fragrance. Some consumers prefer perfume-free compositions.

The perfume of the hair styling composition may be an animal fragrance or a plant fragrance. The animal fragrance may be selected from the group consisting of musk oil, civet, castoreum, ambergris, and mixtures thereof.

The plant fragrance may be selected from the group consisting of nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

The perfume of the composition may be selected from the group consisting of acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitronellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-α-naphthyl ketone, methylphenylcarbinyl acetate, mugetanol, γ-nonalactone, octanal, phenyl ethyl acetate, phenyl-acetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox, and mixtures thereof.

### Styling the hair fibers

The method of the present invention comprises the step of applying the hair styling composition to the hair fibers. The hair styling composition may be applied to dried or wet hair fibers.

For this step, the hair styling composition of the invention may be employed in an application form for a relatively easy, and overall or localized application. Hence, the hair styling composition may be applied to the hair fibers by a an application form selected from the group consisting of a hair styling gel, a non-aerosol hair spray, a hair styling foam, a hair styling wax, a hair styling cream and an aerosol hair spray.

The application form may be preferably a hair styling gel or a non-aerosol hair spray. The hair styling composition employed in an application form as a hair styling gel may be more preferably applied to dry hair fibers. The hair styling composition employed in an application form as a non-aerosol hair spray may be more preferably applied to wet hair fibers. The application of the hair styling composition to the hair fibers is therefore relatively easy. The film-forming polymer deposit formed on the hair fibers can be homogeneous, smooth and can have an excellent adhesion to the hair fibers.

### Hair styling gel

When the hair styling composition is present in the form of a hair styling gel, at least one gel-forming substance is present in it in an amount of preferably from 0.05% to 10%, especially preferably from 0.1% to 2%, by weight of the hair styling composition. The viscosity of the hair styling gel may amount to, preferably, from 0,5 Pa.s to 50 Pa.s, especially preferably from 1 Pa.s to 15 Pa.s at 25°C (measured with a rotary viscometer, RheoStress 100 of Haake at a temperature of 25°C and a shear rate of 0.5 to 1400 s⁻¹).

### Non-aerosol hair spray

When the hair styling composition is in the form of a non-aerosol hair spray, it is sprayed with the help of a suitable mechanically driven spraying device. The term "mechanical spraying devices" means those devices, which permit the spraying of a composition without the use of a propellant. These mechanical spraying devices include a spray pump or an elastic container provided with a spray valve. The hair styling composition is filled under pressure into the elastic container, which stretches so that the hair styling composition will be dispensed when the spray valve is opened so that the elastic container contracts.

### Hair styling foam

When the hair styling composition is in the form of a hair styling foam, at least one common foam-forming substance known for that purpose is included in it. The hair styling composition may be foamed with or without the aid of a propellant gases or chemical propellants and worked into the hair as a foam which loads the hair without being rinsed out. The hair styling composition may be then used with a device for foaming the hair styling composition. Those devices, which permit the foaming of a liquid with or without the use of a propellant, are suitable as foam-forming devices. Suitable mechanical foaming devices can be employed, for example a commercial foam pump or an aerosol foam head.

### Hair styling wax

When the hair styling composition is present in the form of a hair styling wax, it contains one or more water-insoluble fatty or waxy substances, preferably in an amount of from 0.5% to 30% by weight of the hair styling composition, which impart a wax-like consistency to the hair styling composition.

Suitable water-insoluble materials may be, for example, emulsifiers with an HLB-value under 7, silicone oils, silicone waxes, waxes (e.g. waxy alcohol, waxy acids, waxy esters, and especially natural waxes such as beeswax, carnauba wax, etc), fatty alcohol, fatty acids, fatty ester or high molecular weight polyethylene glycols having a molecular weight of 800 to 20,000, preferably from 2,000 to 10,000 g/mol.

### Hair styling cream

When the hair styling composition is present in the form of a hair styling cream, it is preferably an emulsion and additional viscosity imparting substances, in an amount of from 0.1% to 10% by weight of the hair styling composition, which are included in it. Alternatively, the required viscosity and creamy consistency may be built up by micell building with the aid of suitable emulsifiers, fatty acids, fatty alcohols, waxes, etc. in the usual way.

### Aerosol hair spray

The aerosol hair spray may comprise a pressurized container, the hair styling composition which is contained in the pressurized container together with a propellant, and a spraying device connected with the pressurized container for dispensing the hair styling composition from the pressurized container.

When the hair styling composition is present in the form of an aerosol hair spray, it contains from 15% to 85% by weight, preferably from 25% to 75% by weight, of a propellant and is filled in a pressurized container. Lower alkanes, for example, such as n-butane, iso-butane and propane, or their mixtures, dimethyl ether, fluorinated hydrocarbons, such as F-152a (1,1-difluoroethane) or F-134 (tetrafluoroethane) and also propellants present in the form of compressed gases, are suitable as the propellant contained in the aerosol hair spray. Propane and butane may be especially preferred as the propellants. The solvent may be preferably purely alcoholic, but can also contain up to 10% by weight, preferably from 2% to 8%, by weight water.

The hair styling composition may be applied to the hair fibers for a time period ranging from 5 min to 30 min, preferably from 5 min to 15 min, more preferably from 10 to 15 min. After applying the hair styling composition to the hair fibers, the hair fibers may be dried. The hair fibers may be dried with a blow dryer for a time period ranging from 5 min to 30 min, preferably from 5 min to 15 min, more preferably from 10 to 15 min. The period of time may vary according to the concentration of the film-forming polymer in the hair styling composition. The more the film-forming polymer is present in the hair styling composition, the less time it is needed to dry the hair fibers.

The drying may be carried out by a hair dryer, a blow dryer, or a hood device. Hair dryer or blow dryer distances between device and heads may be typically at a distance of 20 or 30 or 40 centimeters, at a temperature between 40°C to 70°C.

Dusting or flaking off from the hair styling composition has not been observed when the hair fibers treated according of the method of the present invention are subjected to stresses such as brushing or combing, or over time.

As disclosed herein, the method of the present invention comprises, after applying the hair styling composition, the step of heating the hair fibers comprising the hair styling composition at a temperature from 80°C to 230°C, preferably from 100°C to 220°C, more preferably from 120°C to 180°C. The heating step may be achieved using a hair dryer, a blow dryer, a hood device or a bonnet hair dryer for example, but also with one of the heating appliance as described hereinafter.

For the heating step, hair dryer or blow dryer distances between device and head may be typically down to 10 centimeters. Blow dryers direct hot hair through some sort of attachment for combing or otherwise treating the hair. The step of heating the hair fibers comprising the hair styling composition may be carried out by a blow dryer at a temperature from 80°C to 130°C, preferably from 100°C to 130°C.

The step of heating may be achieved with a heating appliance selected from the group consisting of a flat iron, a round iron, a curling iron, a straightening iron and a waving iron.

The term "irons" as used herein means a device for heating the hair fibers that brings the hair fibers and the heating device into contact.

Suitable examples of irons that can be used in the method according to the present disclosure, may be all types of flat or round irons and, such as those disclosed in U.S. Pat. Nos. 4,103,145; 4,308,878; 5,983,903; 5,957,140; 5,494,058; and 5,046,516.

In flat irons, the end of the iron that comes into contact with the hair has two planar surfaces. These two planar surfaces may be metallic. They may be smooth or notched.

The application of the heating appliance may be carried out by successive separate touches of a few seconds, or by gradual movement or sliding along the group of hair fibers united by the hair styling composition.

The application of the heating appliance in the method according to the present invention may be carried out by continuous movement from the root to the tip, in at least one pass.

The method of the present invention comprises the step of setting the hair fibers in a hair style without subsequent rinsing.

The hair fibers may be set in the hair style according to a styling step, wherein the styling step comprises an operation chosen from the group selected from shaping, crimping, waving, straightening, curling, and combinations thereof.

Setting the hair fibers in a hair style, may use a tool selected from the group consisting of clamps, rollers, an heating appliance, and combinations thereof.

The step (d) of setting the hair fibers in a hair style is performed subsequently after, simultaneously with or before the step (c) of heating the hair fibers. Finally the hair fibers are cooled to room temperature, i.e. 25°C.

When the step (d) of setting the hair fibers in a hair style is performed subsequently after the step (c) of heating the hair fibers, the hair fibers may be set when the hair fibers are still at a relative hot temperature, preferably from 65°C to 100°C.

When the step (d) of setting the hair fibers in a hair style is performed before subsequently the step (c) of heating the hair fibers, the hairdresser or the consumer may use known styling tools which include, but not limited to: curlers, clamps, rollers, rods of different sizes. After positioning the selected styling tools to the hair fibers coated with the hair styling composition, the hair fibers can be heated using a heating device as set out hereinbefore, such as a hooded dryer, for instance.

When the step (d) of setting the hair fibers in a hair style is performed simultaneously with the step (c) of heating the hair fibers, the hairdresser or the consumer may use different styling and heating tools which can also style the hair at the same time. Suitable styling and heating tools may include, but not limited to: heating rollers. An example of such heating rollers can be the O Rollers supplied by Cloud Nine® USA. The skilled person will know which styling and heating tools he shall use to create the desired shape to the hair fibers coated with the hair styling composition.

Heating and setting hair fibers may be simultaneously achieved using an heating appliance selected from the group consisting of a flat iron, a round iron, a curling iron, a straightening iron and a waving iron.

An homogeneous group of united hair fibers is formed. The cosmetic feel of the resulting styled hair fibers and its softness can be very good. The sheen can also very good. There is also no dusting or no flaking off from the hair styling composition over time or when brushing the hair fibers for instance.

The properties obtained can be durable, as the method does not require reapplication of the hair styling composition over time.

As shown by the Examples hereinafter, the hair style of the hair fibers coated with the hair styling composition can be thermoreversible, that is to say that a new shape may be given to the group of united hair fibers when it is subjected to heat again, without an additional supply of the hair styling composition.

Hence, when the hair style starts to disappear or move, i.e. the group of united hair fibers starts to separate from each other, the method may further comprise an additional step of heating the hair fibers comprising the hair styling composition at a temperature from 80°C to 220°C, preferably from 100°C to 220°C, more preferably from 120°C to 180°C, in order to reach a temperature beyond the glass transition of the film-forming polymer. The film-forming polymer can thus reach a transition state, which allows styling the hair and to form again the group of united hair fibers. The steps of heating, styling and cooling the hair fibers may be repeated as often as necessary to form the desired shape of the hair fibers.

Hence, the method as described herein can make it possible to easily confer a thermoreversible style to the hair fibers treated with the hair styling composition without any needs to add a further amount of the hair styling composition to the hair fibers over time. The hair style may be temporary as the film-forming polymer of the hair styling composition is water-soluble or water-dispersible in water. Hence, after washing the hair fibers with deionized water or with a conventional shampoo composition, the film-forming polymer can be eliminated from the hair fibers.

### Use

A second aspect of the present invention relates to the use of a hair styling composition. The hair styling composition comprises a total amount of film-forming polymer ranging from 2% to 60%, preferably from 5% to 50%, more preferably from 7% to 35%, even more preferably from 10% to 20% by total weight of the hair styling composition. The film-forming polymer comprises one or more polymers, preferably polyesters or acrylic copolymers, selected from block, graft, branched and cross-linked copolymers derived from two or more monomers selected from the group consisting of (meth)acrylic acid, crotonic acid, itaconic acid, citraconic acid, aconitic acid, (C₁-C₄-alkyl)-ester of (meth)acrylic acid, (C₁-C₄-hydroxyalkyl)-ester of (meth)acrylic acid, (meth)acrylamide, N-(C₁-C₄-alkyl)(meth)acrylamide, maleic acid, fumaric acid, aryl-dicarboxylic acid, aryl-acid anhydride, (C₁-C₄-alkyl)-ester of aryl-dicarboxylic acid, aryl-sulfonated dicarboxylic acid, aryl-sulfonated acid anhydride, (C₁-C₄-alkyl)-ester of aryl-sulfonated dicarboxylic acid, and mixtures thereof. The film-forming polymer is water-soluble or water-dispersible at 25°C. The film-forming polymer has a glass transition temperature from -20°C to 100°C, preferably from -20°C to 70°C, more preferably from 0°C to 65°C.

The use of the hair styling composition can help to immobilize the hair temporarily before any subsequent hair treatments, such as hair care treatments. Also or alternatively, the use of the hair styling composition can help to style the hair in a thermoreversible manner. Due to the relatively low glass transition temperature of the film-forming polymer, but also due to the particular adhesion and cohesion properties between the film-forming polymer and the hair fibers, the hair styling composition can help to shape the hair fibers in a thermoreversible manner by heating the hair fibers, and without adding any additional amount of the hair styling composition. Also, as the film-forming polymer is water-soluble or water-dispersible at 25°C, the hair style can be readily removed by washing the hair fibers with a conventional shampoo composition for instance.

### Kit

A third aspect of the present invention is related to a kit for styling hair fibers, preferably human hair fibers, comprising:
- an hair styling composition;
   wherein the hair styling composition comprises a total amount of film-forming polymer ranging from 2% to 60%, preferably from 5% to 50%, more preferably from 7% to 35%, even more preferably from 10% to 20% by total weight of the hair styling composition;
   wherein the film-forming polymer comprises one or more polymers selected from block, graft, branched and cross-linked copolymers derived from two or more monomers selected from the group consisting of acrylic acid, methacrylic acid, crotonic acid, itaconic acid, citraconic acid, aconitic acid, (C₁-C₄-alkyl)-ester of acrylic acid, (C₁-C₄-alkyl)-ester of methacrylic acid, acrylamide, N-(C₁-C₄-alkyl)(meth)acrylamide, maleic acid, fumaric acid, aryl-dicarboxylic acid, aryl-acid anhydride, (C₁-C₄-alkyl)-ester of aryl-dicarboxylic acid, aryl-sulfonated dicarboxylic acid, aryl-sulfonated acid anhydride, (C₁-C₄-alkyl)-ester of aryl-sulfonated dicarboxylic acid, (C₁-C₄-alkyl)-ester of aryl-sulfonated dicarboxylic acid, and mixtures thereof;
   wherein the film-forming polymer is water-soluble or water-dispersible at 25°C;
   wherein the film-forming polymer has a glass transition temperature from -20°C to 100°C; preferably from -20°C to 70°C, more preferably from 0°C to 65°C; preferably in a form of a hair styling gel or a non-aerosol hair spray; and
- an instructional material for preparation, for use, or both preparation and use, of the hair styling composition; and
- optionally an appliance for heating and setting the hair fibers in hair style, wherein the appliance is selected from the group consisting of a flat iron, a round iron, a curling iron, a straightening iron and a waving iron.

The instructional material can contain instructions how to use the hair styling composition to shape the hair fibers according the method of the present invention, particularly that it is possible to set the hair fibers coated with the hair styling composition at will by heating the hair fibers sufficiently with the assistance of a heating appliance or any other sophisticated tools like the O Rollers supplied by Cloud Nine® USA.

### EXAMPLES

The following examples are non-limiting examples of the present invention. The examples are given solely for the purpose of illustration, and are not to be construed as limitations of the present invention since many variations thereof are possible without departing from the spirit and scope of the invention, which would be recognized by one of ordinary skill in the art. All concentrations are listed as weight percent (% wt.), unless otherwise specified. "QSP" means sufficient quantity for 100% or for 100 g. "QS" means sufficient quantity for

### Hair styling compositions Examples C1, C2 and C3

The following hair styling compositions were prepared.

| **Ingredients** | **C1** | **C2** | **C3** |
|---|---|---|---|
| | (% wt.) | (% wt.) | (% wt.) |
| Film-forming polymer | Eastman AQ™ 38S Polymer | Eastman AQ™ 2350 Polymer | Fixate™ Plus Polymer |
| | 15 | 15 | 20 |
| Aminomethyl propanol | 0 | 0 | 1.3 |
| Methylparaben | 0.3 | 0.3 | 0.3 |
| Propylparaben | 0.15 | 0.15 | 0.3 |
| Phenoxyethanol | 0.5 | 0.5 | 0.5 |
| PEG-40 hydrogenated Castor Oil | 1.0 | 1.0 | 1.0 |
| Water purified | QSP | QSP | QSP |

| | | | |
|---|---|---|---|
| Eastman AQ™ 38S Polymer is supplied by Eastman Chemical Company, and is a sulfopolyester, namely Polyester-5 (INCI name); Eastman AQ™ 2350 Polymer is supplied by Eastman Chemical Company, and is another sulfopolyester, also namely Polyester-5 (INCI name); Fixate™ Plus Polymer is supplied by Lubrizol, and is a slightly cross-linked acrylate copolymer of (C₁-C₄ alkyl)(meth-)acrylate, (meth)acrylic acid, namely Polyacrylate-14 (INCI name) | | | |

### Hair styling formulations A-D

The following formulation was prepared:

### Aerosol hair spray

| **Formulation A** | (% wt.) |
|---|---|
| Film-forming polymer | Eastman AQ™ 38S Polymer |
| | 15 |
| Dimethyl ether | 40 |
| Deionised water | QS 100 |
| Perfume and optional ingredients | QSP |

The formulation A was filled in a pressurized aerosol spray can.

The following formulations are prepared:

### Non-aerosol hair spray

| **Formulation B** | (% wt.) |
|---|---|
| Film-forming polymer | Eastman AQ™ 2350 Polymer |
| | 15 |
| Deionised water | QS 100 |
| Perfume and optional ingredients | QSP |

### Hair styling gel

| **Formulation C** | (% wt.) |
|---|---|
| Film-forming polymer | Fixate™ Plus Polymer |
| | 20 |
| Aminomethyl Propanol | 1.3 |
| Preservative | 0.3 |
| Deionised water | QSP 100 |

### Hair styling foam

| **Formulation D** | (% wt.) |
|---|---|
| Film-forming polymer | Eastman AQ™ 2350 Polymer |
| | 15 |
| Gafquat® 755 N, ISP | 0.5 |
| Propane/butane, 2.7 bar | 2 |
| Dimethyl ether | 6 |
| Deionised water | QS 100 |
| Perfume and optional ingredients | QSP |

| | |
|---|---|
| Gafquat® 755 N, ISP is vinyl pyrrolidone/dimethylaminoethyl-methacrylate methosulfate copolymer, 20% in water | |

### Material needed

### Hair strands: Art 811300-9, Color 9/0, Mixture 811

Virgin hair strands having a width of 1.4 cm and a length of 13 cm.
Available from *Kerling International Haarfabric GmbH, Backnang, Germany*
Mass: 1.15 g ± 0.10 g

The following examples according to the present invention have been prepared:

### Example 1

0.30 g of the hair styling composition C1 was applied to a 1.15 g of a blond (9/0) hair strand that had previously been shampooed with Wella™ Professionals Brillance, Fine/Normal shampoo commercially available in Germany in April 2014 and conditioned with Wella™ Professionals Brillance, Fine/Normal conditioner commercially available in Germany in April 2014. The hair strand was dried with a hair dryer for 30 min. The hair strand was heated by being wound around a curling iron at 200°C to obtain ringlets (first heating step).

The same hair strand was then straightened with a flat iron and then recurled with the curling iron. The resulting ringlets were identical to the ones obtained in the first heating step. Hence, the method for styling the hair fibers was thermoreversible. It was possible to recover or to modify the setting of the hair fibers without further adding the hair styling composition C1.

When the hair strand was shampooed with Wella™ Professionals Brillance, for 2 minutes and then blow-dried, almost all polymer residue of the hair styling composition C1 has been removed from hair, which has been acceptable.

### Example 2

0.30 g of the hair styling composition C2 was applied to a 1.15 g of a blond (9/0) hair strand that had previously been shampooed with Wella™ Professionals Brillance, Fine/Normal shampoo commercially available in Germany in April 2014 and conditioned with Wella™ Professionals Brillance, Fine/Normal conditioner commercially available in Germany in April 2014. The hair strand was dried with a hair dryer for 30 min. The hair strand was heated by being wound around a curling iron at 200°C to obtain ringlets (first heating step).

The same hair strand was then straightened with a flat iron and then recurled with the curling iron. The resulting ringlets were identical to the ones obtained in the first heating step. Hence, the method for styling the hair fibers was thermoreversible. It was possible to recover or to modify the setting of the hair fibers without further adding the hair styling composition C1.

When the hair strand was shampooed with Wella™ Professionals Brillance, for 2 minutes and then blow-dried, almost all polymer residue of the hair styling composition C1 has been removed from hair, which has been acceptable.

### Example 3

0.30 g of the hair styling composition C3 was applied to a 1.15 g of a blond (9/0) hair strand that had previously been shampooed with Wella™ Professionals Brillance, Fine/Normal shampoo commercially available in Germany in April 2014 and conditioned with Wella™ Professionals Brillance, Fine/Normal conditioner commercially available in Germany in April 2014. The hair strand dried with a hair dryer for 30 min. The hair strand was heated by being wound around a curling iron at 200°C to obtain ringlets (first heating step).

The same hair strand was then straightened with a flat iron and then recurled with the curling iron. The resulting ringlets were identical to the ones obtained in the first heating step. Hence, the method for styling the hair fibers was thermoreversible. It was possible to recover or to modify the setting of the hair fibers without further adding the hair styling composition C2.

When the hair strand was shampooed with Wella™ Professionals Brillance, for 2 minutes and then blow-dried, all polymer residue of the hair styling composition C2 has been completely removed from hair.

### EXPERIMENTAL

### Viscosity Test Method

The composition may have a kinematic viscosity of from 0,025 Pa.s to 30 Pa.s, measured at 25°C according to the following method. "Viscosity" can mean dynamic viscosity (measured in mPa·s) or kinematic viscosity (measured in centipoises, cPs) of a liquid at 25°C and ambient conditions. Dynamic viscosity may be measured using a rotational viscometer, such as a Brookfield Dial Reading Viscometer Model 1-2 RVT available from Brookfield Engineering Laboratories (USA) or other substitutable model as known in the art. Typical Brookfield spindles which may be used include, without limitation, LV-2 at a spindle speed of 20 rpm, recognizing that the exact spindle may be selected as needed by one skilled in the art. Kinematic viscosity may be determined by dividing dynamic viscosity by the density of the liquid (at 25°C and ambient conditions), as known in the art.

## Claims

1. A method for styling hair fibers, preferably human hair fibers, comprising the following steps:
(a) providing a hair styling composition comprising, in a cosmetically acceptable carrier, a film-forming polymer;
wherein the hair styling composition comprises a total amount of film-forming polymer ranging from 2% to 60%, preferably from 5% to 50%, more preferably from 7% to 35%, even more preferably from 10% to 20% by total weight of the hair styling composition;
wherein the film-forming polymer comprises one or more aryl-sulfonated polyester being a polycondensate of at least one dicarboxylic acid or of an ester thereof, of at least one diol and of at least one sulfoaryldicarboxylic difunctional compound substituted on the aromatic nucleus with a sulfonated group -SO₃M in which M represents a hydrogen atom, an ammonium ion, substituted ammonium or a metal ion such as Na⁺, Li⁺ or K⁺; and wherein the aryl-sulfonated polyester is obtained from isophthalic acid, from the sodium salt of sulfoisophthalic acid, from diethylene glycol and from 1,4-cyclohexane-methanol; or
wherein the film-forming polymer comprises one or more acrylic copolymers are crosslinked acrylic copolymers obtained by polymerization from a monomer composition comprising three different monomer components, a1), b1), c1), and an optional fourth monomer component d1); wherein the first monomer component a1) is (meth)acrylic acid; wherein the second monomer b1) is selected from at least one monoethylenically unsaturated monomer containing at least one linear or branched C₁ to C₄ alkyl ester of (meth)acrylic acid, and mixtures thereof; wherein the third monomer c1) is a mixture of crosslinking monomers from at least two different polyunsaturated monomer types or classes; and wherein the optional fourth monomer d1) is a monoethylenically unsaturated monomer different from monomer components a1), b1), and c1);
wherein the film-forming polymer is water-soluble or water-dispersible at 25°C;
wherein the film-forming polymer has a glass transition temperature from -20°C to 100°C, preferably from -20°C to 70°C, more preferably from 0°C to 65°C;
(b) applying the hair styling composition to the hair fibers;
(c) heating the hair fibers comprising the hair styling composition at a temperature from 80°C to 230°C, preferably from 100°C to 220°C, more preferably from 120°C to 180°C;
(d) setting the hair fibers in a hair style without subsequent rinsing; wherein the step (d) is performed subsequently after, simultaneously with or before the step (c); and
(e) cooling to room temperature.

2. The method for styling hair fibers according to claim 1, wherein the hair styling composition has a viscosity of from 0,025 Pa.s to 30 Pa.s, preferably from 0,05 Pa.s to 3 Pa.s according to the Viscosity Test Method as disclosed herein.

3. The method for styling hair fibers according to any preceding claims, wherein the hair styling composition is substantially free of organic solvents.

4. The method for styling hair fibers according to any preceding claims, wherein the hair styling composition is substantially free of surfactants, or comprises a total amount of surfactants ranging from 1% to 10%, preferably from 1.5% to 5% by total weight of the hair styling composition.

5. The method for styling hair fibers according to any preceding claims, wherein the hair styling composition has a pH from 5 to 8, preferably from 6 to 7.5.

6. The method for styling hair fibers according to any preceding claims, wherein the hair styling composition is applied to the hair fibers by an application form selected from the group consisting of a hair styling gel, a non-aerosol hair spray, a hair styling foam, a hair styling wax, a hair styling cream and an aerosol hair spray.

7. The method for styling hair fibers according to any preceding claims, wherein the hair fibers are set in the hair style according to a styling step, wherein the styling step comprises an operation chosen from the group selected from shaping, crimping, waving, straightening, curling, and combinations thereof.

8. The method for styling hair fibers according to any preceding claims, wherein setting the hair fibers in a hair style, uses a tool selected from the group consisting of clamps, rollers, a heating appliance, and combinations thereof.

9. The method for styling hair fibers according to any preceding claims, wherein heating and setting hair fibers are simultaneously achieved using an heating appliance selected from the group consisting of a flat iron, a round iron, a curling iron, a straightening iron and a waving iron.

10. The method for styling hair fibers according to any preceding claims, wherein the method further comprises an additional step of heating the hair fibers comprising the hair styling composition at a temperature from 80°C to 220°C.

11. Use of a hair styling composition for immobilizing the hair temporarily and/or styling the hair in a thermoreversible manner and temporarily;
wherein the hair styling composition comprises a total amount of film-forming polymer ranging from 2% to 60%, preferably from 5% to 50%, more preferably from 7% to 35%, even more preferably from 10% to 20% by total weight of the hair styling composition;
wherein the film-forming polymer comprises one or more aryl-sulfonated polyester being a polycondensate of at least one dicarboxylic acid or of an ester thereof, of at least one diol and of at least one sulfoaryldicarboxylic difunctional compound substituted on the aromatic nucleus with a sulfonated group -SO₃M in which M represents a hydrogen atom, an ammonium ion, substituted ammonium or a metal ion such as Na⁺, Li⁺ or K⁺; and wherein the aryl-sulfonated polyester is obtained from isophthalic acid, from the sodium salt of sulfoisophthalic acid, from diethylene glycol and from 1,4-cyclohexane-methanol; or wherein the film-forming polymer comprises one or more acrylic copolymers are crosslinked acrylic copolymers obtained by polymerization from a monomer composition comprising three different monomer components, a1), b1), c1), and an optional fourth monomer component d); wherein the first monomer component a1) is (meth)acrylic acid; wherein the second monomer is a linear or branched C₁ to C₄ alkyl ester of (meth)acrylic acid; wherein the third monomer c1) is a mixture of crosslinking monomers; and wherein the optional fourth monomer is a monoethylenically unsaturated monomer different from monomer components a1), b1), and c1);;
wherein the film-forming polymer is water-soluble or water-dispersible at 25°C; and wherein the film-forming polymer has a glass transition temperature from -20°C to 100°C, preferably from -20°C to 70°C, more preferably from 0°C to 65°C.

12. A kit for styling hair fibers, preferably human hair fibers, comprising:
an hair styling composition;
wherein the hair styling composition comprises a total amount of film-forming polymer ranging from 2% to 60%, preferably from 5% to 50%, more preferably from 7% to 35%, even more preferably from 10% to 20% by total weight of the hair styling composition;
wherein the film-forming polymer comprises one or more aryl-sulfonated polyester being a polycondensate of at least one dicarboxylic acid or of an ester thereof, of at least one diol and of at least one sulfoaryldicarboxylic difunctional compound substituted on the aromatic nucleus with a sulfonated group -SO₃M in which M represents a hydrogen atom, an ammonium ion, substituted ammonium or a metal ion such as Na⁺, Li⁺ or K⁺; and wherein the aryl-sulfonated polyester is obtained from isophthalic acid, from the sodium salt of sulfoisophthalic acid, from diethylene glycol and from 1,4-cyclohexane-methanol; or
wherein the film-forming polymer comprises one or more acrylic copolymers are being crosslinked acrylic copolymers obtained by polymerization from a monomer composition comprising three different monomer components, a1), b1), c1), and an optional fourth monomer component d); wherein the first monomer component a1) is (meth)acrylic acid;
wherein the second monomer is a linear or branched C₁ to C₄ alkyl ester of (meth)acrylic acid;
wherein the third monomer c1) is a mixture of crosslinking monomers; and wherein the optional fourth monomer is a monoethylenically unsaturated monomer different from monomer components a1), b1), and c1);
wherein the film-forming polymer is water-soluble or water-dispersible at 25°C; wherein the film-forming polymer has a glass transition temperature from -20°C to 100°C; preferably from -20°C to 70°C, more preferably from 0°C to 65°C;
preferably in a form of a hair styling gel or a non-aerosol hair spray; and
an instructional material for preparation, for use, or both preparation and use, of the hair styling composition; and optionally an appliance for heating and setting the hair fibers in hair style, wherein the appliance is selected from the group consisting of a flat iron, a round iron, a curling iron, a straightening iron and a waving iron.

## Patentansprüche

1. Verfahren zum Stylen von Haarfasern und bevorzugt menschlichen Haarfasern, das die folgenden Schritte umfasst:
(a) Bereitstellen einer Haarstylingzusammensetzung, die in einem kosmetisch akzeptablen Träger ein filmbildendes Polymer umfasst;
wobei die Haarstylingzusammensetzung eine Gesamtmenge an filmbildendem Polymer im Bereich von 2 % bis 60 %, bevorzugt von 5 % bis 50 %, mehr bevorzugt von 7 % bis 35 %, noch mehr bevorzugt von 10 % bis 20 %, des Gesamtgewichts der Haarstylingzusammensetzung umfasst;
wobei das filmbildende Polymer einen oder mehrere arylsulfonierte Polyester umfasst, die ein Polykondensat von mindestens einer Dicarbonsäure oder einem Ester davon, von mindestens einem Diol und von mindestens einer difunktionellen Sulfoaryldicarbonverbindung sind, die an dem aromatischen Kern mit einer sulfonierten Gruppe -SO₃M substituiert ist, wobei M ein Wasserstoffatom, ein Ammoniumion, ein substituiertes Ammonium- oder Metallion, wie beispielsweise Na⁺, Li⁺ oder K⁺, darstellt; und wobei der arylsulfonierte Polyester von Isophthalsäure, von dem Natriumsalz von Sulfoisophthalsäure, von Diethylenglykol und von 1,4 Cyclohexan-methanol erlangt ist; oder
wobei das filmbildende Polymer ein oder mehrere Acrylcopolymere umfasst, die vernetzte Acrylcopolymere sind, die durch die Polymerisation von einer Monomerzusammensetzung erlangt sind, die drei unterschiedliche Monomerkomponenten a1), b1), c1) und eine optionale vierte Monomerkomponente d1) umfasst; wobei die erste Monomerkomponente a1) (Meth)acrylsäure ist; wobei das zweite Monomer b1) von mindestens einem monoethylenisch ungesättigten Monomer ausgewählt ist, das mindestens einen linearen oder verzweigten C₁-C₄-Alkylester von (Meth)acrylsäure und Mischungen davon enthält; wobei das dritte Monomer c1) eine Mischung von vernetzten Monomeren von mindestens zwei unterschiedlichen mehrfach ungesättigten Monomerarten oder - klassen ist; und wobei das optionale vierte Monomer d1) ein monoethylenisch ungesättigtes Monomer ist, das sich von den Monomerkomponenten a1), b1) und c1) unterscheidet;
wobei das filmbildende Polymer bei 25 °C wasserlöslich oder wasserdispergierbar ist;
wobei das filmbildende Polymer eine Glasübergangstemperatur von -20 °C bis 100 °C,
bevorzugt von -20 °C bis 70 °C, mehr bevorzugt von 0 °C bis 65 °C aufweist;
(b) Anwenden der Haarstylingzusammensetzung auf die Haarfasern;
(c) Erwärmen der Haarfasern, welche die Haarstylingzusammensetzung umfassen, auf eine Temperatur von 80 °C bis 230 °C, bevorzugt von 100 °C bis 220 °C, mehr bevorzugt von 120 °C bis 180 °C;
(d) Fixieren der Haarfasern in einer Frisur ohne nachfolgendes Spülen;
wobei der Schritt (d) danach, gleichzeitig mit oder vor Schritt (c) durchgeführt wird; und
(e) Abkühlen auf Raumtemperatur.

2. Verfahren zum Stylen von Haarfasern nach Anspruch 1, wobei die Haarstylingzusammensetzung eine Viskosität von 0,025 Pa.s bis 30 Pa.s, bevorzugt von 0,05 Pa.s bis 3 Pa.s, gemäß dem Viskositätstestverfahren wie hierin offenbart aufweist.

3. Verfahren zum Stylen von Haarfasern nach einem der vorstehenden Ansprüche, wobei die Haarstylingzusammensetzung im Wesentlichen frei von organischen Lösungsmitteln ist.

4. Verfahren zum Stylen von Haarfasern nach einem der vorstehenden Ansprüche, wobei die Haarstylingzusammensetzung im Wesentlichen frei von Tensiden ist oder eine Gesamtmenge an Tensiden im Bereich von 1 % bis 10 %, bevorzugt von 1,5 % bis 5 %, des Gesamtgewichts der Haarstylingzusammensetzung umfasst.

5. Verfahren zum Stylen von Haarfasern nach einem der vorstehenden Ansprüche, wobei die Haarstylingzusammensetzung einen ph-Wert von 5 bis 8, bevorzugt von 6 bis 7,5, aufweist.

6. Verfahren zum Stylen von Haarfasern nach einem der vorstehenden Ansprüche, wobei die Haarstylingzusammensetzung durch eine Anwendungsform auf die Haarfasern aufgebracht wird, die aus der Gruppe bestehend aus einem Haarstylinggel, einem Haarspray ohne Aerosol, einem Haarstylingschaum, einem Haarstylingwachs, einer Haarstylingcreme und einem Aerosolhaarspray ausgewählt wird.

7. Verfahren zum Stylen von Haarfasern nach einem der vorstehenden Ansprüche, wobei die Haarfasern gemäß einem Stylingschritt in die Frisur fixiert werden, wobei der Stylingschritt einen Arbeitsvorgang umfasst, der ausgewählt wird aus der Gruppe bestehend aus Formen, Kräuseln, Wellen, Glätten, Lockenwickeln und Kombinationen davon.

8. Verfahren zum Stylen von Haarfasern nach einem der vorstehenden Ansprüche, wobei beim Fixieren der Haarfasern in einer Frisur ein Werkzeug verwendet wird, das ausgewählt wird aus der Gruppe bestehend aus Klammern, Lockenwicklern, einer Wärmvorrichtung und Kombinationen davon.

9. Verfahren zum Stylen von Haarfasern nach einem der vorstehenden Ansprüche, wobei das Erwärmen und Fixieren von Haarfasern gleichzeitig unter Verwendung einer Wärmvorrichtung erreicht wird, die ausgewählt wird aus der Gruppe bestehend aus einem Flacheisen, einem Rundeisen, einem Lockenstab, einem Glätteisen und einem Welleisen.

10. Verfahren zum Stylen von Haarfasern nach einem der vorstehenden Ansprüche, wobei das Verfahren ferner einen zusätzlichen Schritt des Erwärmens der Haarfasern, welche die Haarstylingzusammensetzung umfassen, bei einer Temperatur von 80 °C bis 220 °C umfasst.

11. Verwendung einer Haarstylingzusammensetzung zum temporären Immobilisieren der Haare und/oder zum Stylen der Haare auf eine thermoreversible Weise und temporär;
wobei die Haarstylingzusammensetzung eine Gesamtmenge an filmbildendem Polymer im Bereich von 2 % bis 60 %, bevorzugt von 5 % bis 50 %, mehr bevorzugt von 7 % bis 35 %, noch mehr bevorzugt von 10 % bis 20 %, des Gesamtgewichts der Haarstylingzusammensetzung umfasst;
wobei das filmbildende Polymer einen oder mehrere arylsulfonierte Polyester umfasst, die ein Polykondensat von mindestens einer Dicarbonsäure oder einem Ester davon, von mindestens einem Diol und von mindestens einer difunktionellen Sulfoaryldicarbonverbindung sind, die an dem aromatischen Kern mit einer sulfonierten Gruppe -SO₃M substituiert ist, wobei M ein Wasserstoffatom, ein Ammoniumion, ein substituiertes Ammonium- oder Metallion, wie beispielsweise Na⁺, Li⁺ oder K⁺, darstellt; und wobei der arylsulfonierte Polyester von Isophthalsäure, von dem Natriumsalz von Sulfoisophthalsäure, von Diethylenglykol und von 1,4 Cyclohexan-methanol erlangt ist; oder
wobei das filmbildende Polymer ein oder mehrere Acrylcopolymere umfasst, die vernetzte Acrylcopolymere sind, die durch die Polymerisation von einer Monomerzusammensetzung erlangt sind, die drei unterschiedliche Monomerkomponenten a1), b1), c1) und eine optionale vierte Monomerkomponente d) umfasst; wobei die erste Monomerkomponente a1) (Meth)acrylsäure ist; wobei das zweite Monomer ein linearer oder verzweigter C₁-C₄-Alkylester von (Meth)acrylsäure ist; wobei das dritte Monomer c1) eine Mischung von vernetzten Monomeren ist; und wobei das optionale vierte Monomer ein monoethylenisch ungesättigtes Monomer ist, das sich von den Monomerkomponenten a1), b1) und c1) unterscheidet;
wobei das filmbildende Polymer bei 25 °C wasserlöslich oder wasserdispergierbar ist; und wobei das filmbildende Polymer eine Glasübergangstemperatur von -20 °C bis 100 °C, bevorzugt von -20 °C bis 70 °C, mehr bevorzugt von 0 °C bis 65 °C aufweist.

12. Kit zum Stylen von Haarfasern und bevorzugt von menschlichen Haarfasern, umfassend:
eine Haarstylingzusammensetzung;
wobei die Haarstylingzusammensetzung eine Gesamtmenge an filmbildendem Polymer im Bereich von 2 % bis 60 %, bevorzugt von 5 % bis 50 %, mehr bevorzugt von 7 % bis 35 %, noch mehr bevorzugt von 10 % bis 20 %, des Gesamtgewichts der Haarstylingzusammensetzung umfasst;
wobei das filmbildende Polymer einen oder mehrere arylsulfonierte Polyester umfasst, die ein Polykondensat von mindestens einer Dicarbonsäure oder einem Ester davon, von mindestens einem Diol und von mindestens einer difunktionellen Sulfoaryldicarbonverbindung sind, die an dem aromatischen Kern mit einer sulfonierten Gruppe -SO₃M substituiert ist, wobei M ein Wasserstoffatom, ein Ammoniumion, ein substituiertes Ammonium- oder Metallion, wie beispielsweise Na⁺, Li⁺ oder K⁺, darstellt; und wobei der arylsulfonierte Polyester von Isophthalsäure, von dem Natriumsalz von Sulfoisophthalsäure, von Diethylenglykol und von 1,4 Cyclohexan-methanol erlangt ist; oder
wobei das filmbildende Polymer ein oder mehrere Acrylcopolymere umfasst, die vernetzte Acrylcopolymere sind, die durch die Polymerisation von einer Monomerzusammensetzung erlangt sind, die drei unterschiedliche Monomerkomponenten a1), b1), c1) und eine optionale vierte Monomerkomponente d) umfasst; wobei die erste Monomerkomponente a1) (Meth)acrylsäure ist; wobei das zweite Monomer ein linearer oder verzweigter C₁-C₄-Alkylester von (Meth)acrylsäure ist;
wobei das dritte Monomer c1) eine Mischung von vernetzten Monomeren ist; und wobei das optionale vierte Monomer ein monoethylenisch ungesättigtes Monomer ist, das sich von den Monomerkomponenten a1), b1) und c1) unterscheidet;
wobei das filmbildende Polymer bei 25 °C wasserlöslich oder wasserdispergierbar ist; wobei das filmbildende Polymer eine Glasübergangstemperatur von -20 °C bis 100 °C, bevorzugt von -20 °C bis 70 °C, mehr bevorzugt von 0 °C bis 65 °C aufweist; bevorzugt in Form eines Haarstylinggels oder eines Haarsprays ohne Aerosol; und
ein Lehrmaterial zur Herstellung, zur Verwendung oder sowohl zur Herstellung als auch zur Verwendung der Haarstylingzusammensetzung; und optional eine Vorrichtung zum Erwärmen und Fixieren der Haarfasern in eine Frisur, wobei die Vorrichtung ausgewählt ist aus der Gruppe bestehend aus einem Flacheisen, einem Rundeisen, einem Lockenstab, einem Glätteisen und einem Welleisen.

## Revendications

1. Procédé de coiffage de fibres capillaires, de préférence de fibres capillaires humaines, comprenant les étapes suivantes :
(a) la fourniture d'une composition de coiffage comprenant, dans un support cosmétiquement acceptable, un polymère filmogène ;
dans lequel la composition de coiffage comprend une quantité totale de polymère filmogène allant de 2 % à 60 %, de préférence de 5 % à 50 %, de préférence de 7 % à 35 %, encore plus de préférence de 10 % à 20 % par poids total de la composition de coiffage ;
dans lequel le polymère filmogène comprend un ou plusieurs polyester aryl-sulfoné étant un polycondensat d'au moins un acide dicarboxylique ou d'un ester de celui-ci, d'au moins un diol et d'au moins un composé difonctionnel sulfoaryldicarboxylique substitué sur le noyau aromatique avec un groupe sulfoné -SO₃M dans lequel M représente un atome d'hydrogène, un ion d'ammonium, un ammonium substitué ou un ion métallique tel que Na⁺, Li⁺ ou K⁺; et dans lequel le polyester aryl-sulfoné est obtenu à partir d'acide isophthalique, de sel de sodium d'acide sulfoisophtalïque, de diéthylène glycol et de 1,4-cyclohexane-méthanol ; ou
dans lequel le polymère filmogène comprend un ou plusieurs copolymères acryliques qui sont des copolymères acryliques réticulés obtenus par polymérisation à partir d'une composition monomère comprenant trois composants monomères différents, a1), b1), c1) et un quatrième composant monomère optionnel d1), dans lequel le premier composant monomère a1) est un acide (meth)acrylique ; dans lequel le deuxième monomère b1) est choisi parmi au moins un monomère monoéthylénique insaturé contenant au moins un ester alkyle C₁ à C₄ linéaire ou ramifié d'acide (meth)acrylique, et le mélange de ceux-ci ; dans lequel le troisième monomère c1) est un mélange de monomères réticulants à partir d'au moins deux types de monomères polyinsaturés différents ; et dans lequel le quatrième monomère optionnel d1) est un monomère monoéthylène insaturé différent des composants monomères a1), b1) et c1) ;
dans lequel le polymère filmogène est hydrosoluble ou hydrodispersable à 25 °C ;
dans lequel le polymère filmogène a une température de transition vitreuse de -20 °C à 100 °C, de préférence de -20 °C à 70 °C, plus de préférence de 0 °C à 65 °C ;
(b) l'application d'une composition de coiffage sur les fibres capillaires ;
(c) le chauffage des fibres capillaires comprenant la composition de coiffage à une température de 80 °C à 230 °C, de préférence de 100 °C à 220 °C, plus de préférence de 120 °C à 180 °C ;
(d) la fixation des fibres capillaires dans un coiffage sans rinçage ultérieur ;
dans lequel l'étape (d) est réalisée par la suite après, simultanément à ou avant l'étape (c) ; et
(e) le refroidissement à température ambiante.

2. Procédé de coiffage de fibres capillaires selon la revendication 1, dans lequel la composition de coiffage a une viscosité de 0,025 Pa.s à 30 Pa.s, de préférence de 0,05 Pa.s à 3 Pa.s selon le procédé de test de viscosité tel que décrit ici.

3. Procédé de coiffage de fibres capillaires selon l'une quelconque des revendications précédentes, dans lequel la composition de coiffage est sensiblement exempte de solvants organiques.

4. Procédé de coiffage de fibres capillaires selon l'une quelconque des revendications précédentes, dans lequel la composition de coiffage est sensiblement exempte de surfactants, ou comprend une quantité totale de surfactants allant de 1 % à 10 %, de préférence de 1,5 % à 5 % par poids total de la composition de coiffage.

5. Procédé de coiffage de fibres capillaires selon l'une quelconque des revendications précédentes, dans lequel la composition de coiffage a un pH de 5 à 8, de préférence de 6 à 7,5.

6. Procédé de coiffage de fibres capillaires selon l'une quelconque des revendications précédentes, dans lequel la composition de coiffage est appliquée aux fibres capillaires par une forme d'application choisie parmi le groupe constitué d'un gel coiffant, d'une laque non aérosol, d'une mousse coiffante, d'une cire coiffante, d'une crème coiffante et d'une laque aérosol.

7. Procédé de coiffage de fibres capillaires selon l'une quelconque des revendications précédentes, dans lequel les fibres capillaires sont définies dans le coiffage selon une étape de coiffage, dans laquelle l'étape de coiffage comprend une opération choisie dans le groupe sélectionné parmi la mise en forme, le frisage, l'ondulation, le lissage, le bouclage, et les combinaisons de ceux-ci.

8. Procédé de coiffage de fibres capillaires selon l'une quelconque des revendications précédentes, dans lequel la définition des fibres capillaires dans un coiffage, utilise un outil choisi parmi le groupe constitué de pinces, rouleaux, appareils d'application de chauffage et des combinaisons de ceux-ci.

9. Procédé de coiffage de fibres capillaires selon l'une quelconque des revendications précédentes, dans lequel le chauffage et la définition des fibres capillaires sont simultanément obtenus à l'aide d'un appareil de chauffage choisi parmi le groupe constitué d'un fer à lisser, d'une brosse soufflante rotative, d'un fer à boucler, d'une brosse lissante et d'un fer à onduler.

10. Procédé de coiffage de fibres capillaires selon l'une quelconque des revendications précédentes, dans lequel le procédé comprend en outre une étape supplémentaire de chauffage des fibres capillaires comprenant la composition de coiffage à une température de 80 °C à 220 °C.

11. Utilisation d'une composition de coiffage pour l'immobilisation temporaire des cheveux et/ ou le coiffage des cheveux de manière thermoréversible et temporairement ; dans lequel la composition de coiffage comprend une quantité totale de polymère filmogène allant de 2 % à 60 %, de préférence de 5 % à 50 %, de préférence de 7 % à 35 %, encore plus de préférence de 10 % à 20 % par poids total de la composition de coiffage ;
dans lequel le polymère filmogène comprend un ou plusieurs polyester aryl-sulfoné étant un polycondensat d'au moins un acide dicarboxylique ou d'un ester de celui-ci, d'au moins un diol et d'au moins un composé difonctionnel sulfoaryldicarboxylique substitué sur le noyau aromatique avec un groupe sulfoné -SO₃M dans lequel M représente un atome d'hydrogène, un ion d'ammonium, un ammonium substitué ou un ion métallique tel que Na⁺, Li⁺ ou K⁺; et dans lequel le polyester aryl-sulfoné est obtenu à partir d'acide isophthalique, de sel de sodium d'acide sulfoisophtalïque, de diéthylène glycol et de 1,4-cyclohexane-méthanol ; ou
dans lequel le polymère filmogène comprend un ou plusieurs copolymères acryliques qui sont des copolymères acryliques réticulés obtenus par polymérisation à partir d'une composition monomère comprenant trois composants monomères différents, a1), b1), c1) et un quatrième composant monomère optionnel d), dans lequel le premier composant monomère a1) est un acide (meth)acrylique ; dans lequel le deuxième monomère est choisi parmi au moins un monomère monoéthylénique insaturé contenant au moins une ester alkyle C₁ à C₄ linéaire ou ramifié d'acide (meth)acrylique, et le mélanges de ceux-ci ; dans lequel le troisième monomère c1) est un mélange de monomères réticulants à partir d'au moins deux types de monomères polyinsaturés différents ; et dans lequel le quatrième monomère optionnel est un monomère monoéthylène insaturé différent des composants monomères a1), b1) et c1) ;
dans lequel le polymère filmogène est hydrosoluble ou hydrodispersable à 25 °C ; et dans lequel le polymère filmogène a une température de transition vitreuse de -20 °C à 100 °C, de préférence de -20 °C à 70°C, plus de préférence de 0 °C à 65 °C.

12. Kit pour le coiffage de fibres capillaires, de préférence de fibres capillaires humaines, comprenant :
une composition de coiffage ;
dans lequel la composition de coiffage comprend une quantité totale de polymère filmogène allant de 2 % à 60 %, de préférence de 5 % à 50 %, de préférence de 7 % à 35 %, encore plus de préférence de 10 % à 20 % par poids total de la composition de coiffage ;
dans lequel le polymère filmogène comprend un ou plusieurs polyester aryl-sulfoné étant un polycondensat d'au moins un acide dicarboxylique ou d'un ester de celui-ci, d'au moins un diol et d'au moins un composé difonctionnel sulfoaryldicarboxylique substitué sur le noyau aromatique avec un groupe sulfoné -SO₃M dans lequel M représente un atome d'hydrogène, un ion d'ammonium, un ammonium substitué ou un ion métallique tel que Na⁺, Li⁺ ou K⁺; et dans lequel le polyester aryl-sulfoné est obtenu à partir d'acide isophthalique, de sel de sodium d'acide sulfoisophtalïque, de diéthylène glycol et de 1,4-cyclohexane-méthanol ; ou
dans lequel le polymère filmogène comprend un ou plusieurs copolymères acryliques qui sont des copolymères acryliques réticulés obtenus par polymérisation à partir d'une composition monomère comprenant trois composants monomères différents, a1), b1), c1) et un quatrième composant monomère optionnel d), dans lequel le premier composant monomère a1) est un acide (meth)acrylique ; dans lequel le deuxième monomère est un ester alkyle C₁ à C₄ linéaire ou ramifié d'acide (meth)acrylique ; dans lequel le troisième monomère c1) est un mélange de monomères réticulants ; et dans lequel le quatrième monomère optionnel est un monomère monoéthylène insaturé différent des composants monomères a1), b1) et c1) ;
dans lequel le polymère filmogène est hydrosoluble ou hydrodispersable à 25 °C ; dans lequel le polymère filmogène a une température de transition vitreuse de -20 °C à 100 °C, de préférence de -20 °C à 70 °C, plus de préférence de 0 °C à 65 °C ;
de préférence sous forme de gel coiffant ou de laque non aérosol ; et
un matériau pédagogique pour la préparation, pour l'utilisation, ou à la fois la préparation et l'utilisation, de la composition de coiffage ; et optionnellement un appareil pour le chauffage et la définition des fibres capillaires dans un coiffage, dans lequel l'appareil est choisi parmi le groupe constitué d'un fer à lisser, d'une brosse soufflante rotative, d'un fer à boucler, d'une brosse lissante et d'un fer à onduler.
